# EUROPÄISCHE PATENTSCHRIFT

(11) **EP 1 575 468 B1**
(45) Veröffentlichungstag und Bekanntmachung des Hinweises auf die Patenterteilung: **02.03.2011**
(21) Anmeldenummer: 03756483.8
(22) Anmeldetag: 24.10.2003
(51) Int. Cl.: A61F 13/00, A61L 15/20, A61L 15/42, C08K 13/02

(54) **ANTIADHÄSIVE BESCHICHTUNG ZUR AUSRÜSTUNG VON WUNDVERBÄNDEN**
ANTI-ADHESIVE COATING FOR PLASTERS
REVETEMENT ANTI-ADHESIF DESTINE A DES PANSEMENTS

(30) Priorität: 25.10.2002 DE 10249874
(43) Veröffentlichungstag der Anmeldung: 21.09.2005
(73) Patentinhaber: Paul Hartmann Aktiengesellschaft, 89522 Heidenheim (DE)
(72) Erfinder: LANGEN, Günter, 67752 Wolfstein (DE); MEISTER, Marita, 67657 Kaiserslautern (DE); BÖTTCHER, Horst, 01169 Dresden (DE); MAHLTIG, Boris, 01097 Dresden (DE)
(74) Vertreter: Katzameyer, Michael
(86) Internationale Anmeldenummer: PCT/EP2003/011819
(87) Internationale Veröffentlichungsnummer: WO 2004/037139

(56) Entgegenhaltungen:
- EP-A- 0 047 492
- DE-A- 10 006 125
- DE-A- 10 015 600
- DE-A- 10 054 119

## Beschreibung

Die Erfindung betrifft Beschichtungszusammensetzungen zur antiadhäsiven Beschichtung von Wundauflagen, antiadhäsive Schichten für Wundauflagen, antiadhäsiv beschichtete Wundauflagen und Verfahren zur antiadhäsiven Beschnichtung eine Wundauflage.

Die Aufgabe eines Wundverbandes ist, die Wunde vor Umgebungseinflüssen zu schützen, die Wundflüssigkeit aufzunehmen und den Heilungsprozess zu fördern. Es ist bekannt, dass es während des Heilungsprozesses zu einem Verkleben von Verbandmaterial und Wunde kommt. Diese Haftung von Wunde zu Verband kann beim Wechseln der Wundauflage zur erneuten Schädigung der verheilenden Wunde führen. Neben den Schmerzen, welcher ein Patient, insbesondere mit chronischen Wunden, bei regelmäßigen Verbandswechseln zu erleiden hat, führt eine wiederholte Schädigung der heilenden Wunde auch zu einer Verzögerung des Wundheilungsprozesses insgesamt. Aus diesem Grund ist es wünschenswert, dass Wundverbände eine geringe Verklebungsneigung zur Wunde aufweisen.

Bisherige Verfahren zur Erzeugung von Wundverbänden mit geringer Verklebungsneigung gehen zumeist von einem mehrlagigen Verbandsmaterial bestehend aus Schichten verschiedener Textiltypen aus (WO99/611077; EP1097682A2; DE10014557A1). Die Textillage, welche direkt auf der Wunde aufliegt, besteht bei diesen Verbänden zumeist aus einem hydrophoben Textil oder einer Polymerfolie, die nur eine geringe Verklebungsneigung zur Wunde aufweisen. Hinter dieser Kontaktauflage ist dann ein saugfähiges textiles Material zur Aufnahme der Wundflüssigkeit aufgebracht. Solche mehrlagigen Verbandsysteme haben im Gegensatz zu Wundauflagen, bestehend aus nur einem Textiltyp, zwei wesentliche Nachteile. Zum einen ist die Handhabbarkeit weniger komfortabel, da die Wunde ausschließlich mit der Kontaktfläche der Wundauflage bedeckt werden darf. Zum anderen ist die Herstellung solcher mehrlagigen Wundauflagen deutlich kostenintensiver im Vergleich zu Einkomponentenmaterialien, da zwei unterschiedliche textile Materialien in einem weiteren Arbeitsschritt miteinander verbunden werden müssen.

Bei einem neuartigen Typ von mehrlagigen Verbandsmaterial besteht die Oberfläche, welche direkt auf der Wunde aufliegt, aus einer elastomeren Hydrogelschicht, die sowohl hydrophile wie auch hydrophobe Eigenschaften aufweisen kann (WO00/16725; EP261167). Diese Systeme weisen zwar eine deutlich verminderte Verklebungsneigung zur Wunde auf, sind allerdings schwierig in der Handhabung, da zum einen nur die Kontaktauflage selbst auf der Wunde aufliegen darf und zum anderen Vorkehrungen getroffen werden müssen, damit das Hydrogel nicht austrocknet oder verunreinigt wird. Ein weiterer Nachteil ist, dass die Herstellung solcher mehrlagigen Wundverbände mit Hydrogelschicht deutlich kostenintensiver ist als die Produktion von Verbandsystemen bestehend nur aus einem textilen Material.

Aufgrund der Nachteile mehrlagiger Verbandsysteme wurden Verfahren entwickelt, um textile Materialien mit niedriger Verklebungsneigung bei gleichzeitig gutem Wasseraufnahmevermögen zu entwickeln. Ein Verfahren besteht darin, hydrophobe und hydrophile Fäden so miteinander zu verweben, dass eine einlagige textile Wundauflage mit verminderter Wundhaftung entsteht (EP1106149A2). Somit liegt hier ein einlagiger Wundverband aus einem Mischgewebe vor. Günstiger ist hingegen, wenn ein herkömmliches Verbandsmaterial so modifiziert wird, dass es eine verminderte Wundhaftung aufweist.

Ein solches Verfahren stellt die hydrophobe Modifizierung von Cellulose-Material mittels Carboxylierung dar (WO 00/01425). Die Hydrophobierung der Cellulose vermindert die Haftung von Wundauflage zur Wunde und induziert somit eine verminderte Verklebungsneigung. Der Nachteil dieses Verfahrens ist allerdings, dass es im wesentlichen nur die Modifizierung von Celluloseverbänden erlaubt, dagegen andere Verbandsmaterialien wie z.B. Polyamid oder Viskose nicht durch Carboxylierung verbessert werden können.

EP-A-0047492 offenbart antiadhäsive Wundauflagen, bei denen Gewebe bzw. Vliese mit Silikonöl, Paraffin bzw. Vaseline beschichtet werden. In den Druckschriften DE-A-10006125, DE-A-10015600 und DE-A-10054119 werden SiO₂-Nanosole bzw. die entsprechenden Xerogele beschrieben und DE-A-10015600 offenbart deren mögliche Verwendung als Beschichtungsmaterial für Textilien, jedoch geben diese Dokumente keinen Hinweis darauf, dass diese Zusammensetzungen für Wundauflagen mit antiadhäsiven Eigenschaften nützlich sind.

Eine Aufgabe der Erfindung ist es, verbesserte Beschichtungszusammensetzungen zur antiadhäsiven Ausrüstung von Wundauflagen, mit der die Nachteile herkömmlicher Wundauflagen vermieden werden, und Verfahren zur deren Herstellung bereitzustellen. Die Beschichtungszusammensetzungen sollen insbesondere die Herstellung von Wundauflagen mit verminderter Verklebungsneigung zwischen Wunde und Verbandsmaterial ermöglichen. Des weiteren soll eine kostengünstige antiadhäsive Ausrüstung von Wundauflagen ermöglicht werden. Die Erfindung soll ferner bei verschiedenartigen Verbandmaterialien anwendbar sein.

Diese Aufgaben werden durch den Verbund aus eine Beschichtungszusammensetzung oder einer antiadhäsiven Schicht und einer Wundauflage und die Verfahren und Verwendungen, mit den Merkmalen gemäß Anspruch 1, 5, order 9 gelöst. Vorteilhafte Ausführungsformen und Anwendungen der Erfindung ergeben sich aus den abhängigen Ansprüchen.

Eine Grundidee der Erfindung ist es insbesondere, antiadhäsive Beschichtungszusammensetzungen (Beschichtungsmittel) zur Ausrüstung von Wundauflagen auf Basis schichtbildender Nanosole bereitzustellen, die Siliziumoxid und mindestens eine hydrophob oder oleophob wirkende organische Siliziumverbindung (sog. Komponente A) enthalten. Diese Kombination besitzt den besonderen Vorteil, das die Fähigkeit zur Bildung stabiler Schichten, die an der Wundauflage haften, und die antiadhäsive Wirkung der organischen Siliziumverbindung kombiniert werden.

Antiadhäsive Schichten sind hier auf dem als Träger wirkenden Verbandmaterial (oder Wundauflage) gebildete Schichten mit einer

Wirkung, die ein Anhaften von angrenzenden Materialien, insbesondere von Teilen der Wunde, aus dieser austretenden Substanzen, oder auf dieser aufgebrachten Substanzen vermindert.

Die Wundauflage besteht aus einem textilen Flächengebilde, einem Schaumstoff oder einem Gel. Als textiles Flächengebilde (textiles Material) können verschiedene Textiltypen (Gewebe, Gewirke, Gestrick, Non-Woven-Textilien) aus Naturfasern (wie z. B. Baumwolle, Viskose) oder Chemiefasern (wie z. B. Polyamid, Polyester, Polypropylen, Polyethylen, Polyacrylnitril, Polyacetat, Polyurethan, Gummi, Calciumalginat, Chitosan oder Mischungen aus diesen Fasern) verwendet werden. Schaumstoff-Wundauflagen bestehen bspw. aus Naturlatex, Syntheselatex oder Polyurethan. Gele als Wundauflagen bestehen bspw. aus Gelatine, Alginat, Polysacharin, Stärke, Stärkeether, Stärkeester, Cellulose, Celluloseether, Celluloseester, Galaktomannanen oder Polyurethanen und enthalten ggf. Zusätze von Superabsorbern (SOP) auf der Basis von Polyacrylat, Stärkederivaten oder Cellulosederivaten. Die Wundauflage besteht vorzugsweise aus einem einlagigen Material, wie es von herkömmlichen Wundverbände an sich bekannt ist und das neben der verminderten Haftung zur Wunde eine genügende Aufnahmefähigkeit z. B. für Wundsekret aufweist.

Es ist insbesondere vorgesehen, zur Beschichtung von Wundauflagen schichtbildende Nanosole zu verwenden, die durch Hydrolyse von Tetraalkoxysilanen und einer hydrophob und ggf. oleophob wirkenden organischen Siliziumverbindung in organischen, organisch-wässrigen oder wäßrigen Lösemitteln gebildet werden. Mit Hilfe eines Sol-Gel Verfahrens werden auf einer Wundauflage dünne, antiadhäsive quasi-keramische Schichten erzeugt.

Das flüssige Beschichtungsmittel zur Ausrüstung von Wundverbänden umfasst ein hydrophob modifiziertes Siliziumoxid-Nanosol in organischen, wässrigen oder gemischten Lösemitteln. Die Nanosol-Partikel werden dabei durch saure oder alkalisch katalysierte Hydrolyse eines Tetraalkoxysilans Si(OR)₄, wobei R vorzugsweise 1 bis 4 Kohlenstoffatome enthält, hergestellt:

Si(OR)₄ + 2 H₂O ----> (SiO₂)ₙ + 4 ROH (1)

Das gebildete (SiO₂)ₙ liegt in der wässrig-alkoholischen Lösung in nanopartikulärer Form vor. Die mittlere Teilchengröße liegt je nach Reaktionsbedingungen z. B. zwischen 2 und 15 nm. Der bei der Hydrolyse gebildete Alkohol kann schonend verflüchtigt und durch Wasser substituiert werden, so dass bei Bedarf auch rein wässrige Nanosole eingesetzt werden können. Durch das extreme Verhältnis von Teilchenoberfläche zu Teilchenvolume führt eine Veränderung der Umgebungsbedingungen (Neutralisation der Lösung, Temperaturerhöhung, Konzentrationserhöhung) zu einer schnellen Gelierung ("Sol-Gel-Prozess") der Nanosole. Man kann darum Nanosole vorteilhaft als Beschichtungsmittel einsetzen, wobei im Falle einer Beschichtung und nachfolgenden Trocknung erst lösungsmittelhaltige Lyogel-Schichten gebildet werden, die im weiteren Verlauf der Trockung in trockene stabile Xerogel-Schichten übergehen, die einen quasi-keramischen Charakter haben.

SiO₂)ₙ, sol ------> Lyogel ----> Xerogel quasi-keramische Beschichtung (2)

Das relative Beschichtungsgewicht der Xerogelschichten auf der Wundauflage liegt im Bereich von 0.05% bis 5%, vorzugsweise von 0.2% bis 2%. Bei beschichteten Textilien werden je nach Verbindungstyp und Temperungstemperatur bspw. Beschichtungen mit einer Flächendichte von 0.1 g/m² bis 50 g/m² bezogen auf die Fläche des beschichteten Textils gebildet.

Die Beschichtung kann vorteilhafterweise als homogene isotrope Verteilung in der gesamten Wundauflage (z. B. durch Imprägnieren, Tauchbeschichtung, Foulardieren), einseitige Beschichtung als Dünnschicht an einer Oberfläche der Wundauflage (z. B. durch Pflatschen, Walzenauftrag, Sprühbeschichtung, Streichbeschichtung, insbesondere Walzenrakel, Luftrakel) oder als zweiseitige Beschichtung mit Dünnschichten auf beiden Oberflächen der Wundauflage durch gleichzeitig oder nacheinander ausgeführte Schritte eines Auftragsverfahrens wie bei der einseitigen Beschichtung) gebildet werden. Die Beschichtung kann als geschlossene Schicht oder als teilweise offene Schicht, z. B. mit einem netz- oder inselförmigen Muster gebildet werden. Eine derartig gemusterte Dünnschicht wird bspw. durch einen Rasterwalzenauftrag, Schablonendruck oder Siebdruck hergestellt, wobei das jeweils gewünschte geometrische Muster mit der partiellen Verteilung des Nanosols auf der Oberfläche der Wundauflage realisiert wird.

Beschichtet man textile Wundauflagen mit reinen SiO₂-Nanosolen, zeigen die gebildeten SiO₂-Beschichtunge nur geringe Änderungen im adhäsiven Verhalten. Die verminderte Verklebungsneigung der beschichteten Wundauflage wird erfindungsgemäß durch die Zugabe und ggf. Cohydrolyse der hydrophob wirkenden siliziumhaltigen Komponente (A) erzielt. Die Komponente A kann sowohl vor oder auch nach der Hydrolyse des Tetraalkoxysilans entsprechend (1) zugesetzt werden, ohne dass signifikante Eigenschaftsänderungen der Beschichtungslösungen auftreten.

Als Komponente A (organische Siliziumverbindung) können verwendet werden:
- Trialkoxysilan R¹Si(OR)₃, wobei R¹ ein Alkylrest mit 8 bis 18 Kohlenstoffatomen ist. Zunehmende hydropobe Eigenschaften und eine verminderte Wundhaftung lassen sich mit höherer Kettenlänge des Alkylrestes einstellen.
- Arylsilane R²Si(OR)₃ oder Diarylsilane R²₂Si (OR) ₂, wobei R² ein Arylrest, vorzugsweise ein Phenylrest, ist. Der Phenylrest mit 6 C-Atomen bewirkt hier eine Hydrophobierung entsprechend der eines Alkylrests gleicher Länge.
- Triphenylsilanchlorid oder t-Butyldiphenylsilanchlorid. Diese verbessern die Hydrophobierung im Vergleich zu den vorher genannten Arylsilanen.
- Polysiloxane mit Methyl- und/oder Phenylseitengruppen, welche durch reaktive Kettenenden cohydrolysiert werden können. Als reaktive Kettenenden können eingesetzt Hydroxyeinheiten (a) oder Epoxyeinheiten (b) werden.

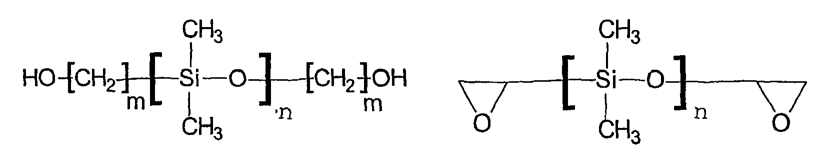

Mit Zunahme des Polymerisationsgrades des Polysiloxans kann eine zunehmende Hydrophobierung der textilen Wundauflage eingestellt werden.
- Alkyltrialkoxysilanverbindungen R³Si(OR)₃ mit einem perfluorierten Alkylrest R³.
- Polysiloxan-Verbindungen mit perfluorierten Alkylseitenketten. Durch das Einbringen von fluorierten Alkylgruppen können auf der beschichteten Wundauflage sowohl hydrophobe wie auch oleophobe Eigenschaften erzielt werden. Die Haftung von beschichteter Wundauflage und Wunde wird entsprechend reduziert.

Neben dem Einsatz von Alkyltrialkoxysilanen R¹Si(OR)₃ als hydrophobe Additive zu reinen SiO₂-Nanosolen können diese Alkylsilan-Verbindungen auch für sich, d. h. ohne Zugabe von TEOS, miteinander in Alkohol hydrolysiert und als Beschichtungsmittel eingesetzt werden.

Die zugesetzte Menge A beträgt in Abhängigkeit vom Verbindungstyp zwischen 1 bis 50 Gew-% bezogen auf die gesamte Feststoff-Menge.

Durch den Zusatz und die Cohydrolyse von Epoxysilanen, vorzugsweise von 3-Glycidyloxypropyl-trialkoxysilanen, in Mengen bis zu 50 Gew-% zu den Tetraalkoxy-Silanen entsprechend Gl. (1), können partiell hydrophile Schichteigenschaften erzielt werden. Diese Eigenschaften fördern bei guter antiadhäsiver Wirkung in vorteilhafter Weise hohe Wasseraufnahmefähigkeit bzw. Wasserdurchlässigkeit des textilen Körpers und damit die Aufnahme des Wundsekretes. Außerdem wird die Haftung der Beschichtung zum textilen Träger verbessert.

Generell bewirkt der Einsatz von hydrolysefähigen, siliziumhaltigen Verbindungen als hydrophobe Komponente A die feste Einbindung der hydrophoben Eigenschaft an die Textilbeschichtung, und stellt somit einen wichtigen Vorteil gegenüber dem Einsatz der reinen Komponenten dar.

Die erfindungsgemäß verwendeten antiadhäsiven Beschichtungen können darum vorteilhaft zur Ausrüstung von Wundauflagen eingesetzt werden, um die Adhäsion zwischen Wunde und Wundauflage zu verringern.

Wichtige Vorteile der Erfindung bestehen gegenüber dem Stand der Technik in folgendem:
◆ Die Haftung zwischen Wunde und Wundauflage wird vermindert.
◆ Die eingesetzten Beschichtungsmittel erlauben die Übertragung der nichthaftenden Eigenschaften auf herkömmliche insbesondere einlagige textile Wundauflagen, während bisher meist mehrlagige Auflagen aus verschiedenen textilen Materialien eingesetzt wurden.
◆ Die nichthaftenden Eigenschaften können mit Hilfe des Beschichtungsmittels auf verschiedene Textiltypen gleichermaßen aufgetragen werden.
◆ Die zusätzliche Beigabe hydrophiler Komponenten ermöglicht die Herstellung textiler Materialien mit hydrophober Oberfläche und verminderter Verklebungsneigung bei gleichzeitig vorteilhaften Flüssigkeitsaufnahmevermögen des textilen Substrates.

### Ausführungsbeispiele

### 1. Herstellung der Nanosole

Sol 1: Zu einer Mischung aus 20ml Tetraethoxysilan (TEOS) und 84ml Ethanol werden 4ml 0,01N HCl tropfenweise zugesetzt und 20 Stunden bei Raumtemperatur gerührt. Es entsteht ein wasserklares stabiles Sol.

Sol 2: Zu einer Mischung aus 34ml TEOS, 4ml 3-Glycidyloxypropyltriethoxysilan (GLYEO), 50ml Ethanol werden 12ml 0,01 N HCl tropfenweise zugesetzt und 20 Stunden bei Raumtemperatur gerührt. Es entsteht ein wasserklares stabiles Sol.

Sol3: Zu einer Mischung aus 5ml TEOS, 15ml Methyltriethoxysilan, 85ml EtOH werden 5ml 0,01 N HCl tropfenweise zugesetzt und 20 Stunden bei Raumtemperatur gerührt. Es entsteht ein wasserklares stabiles Sol.

Sol4: Eine Mischung aus 15ml Methyltriethoxysilan und 15ml Phenyltriethoxysilan in 90ml Ethanol und 5 ml 0.01N HCl wird 20 Stunden bei Raumtemperatur gerührt.

So15: Zu einer Mischung aus 20ml Methyltriethoxysilan und 85ml Ethanol werden 5ml 0,01 N HCl tropfenweise zugesetzt und 20 Stunden bei Raumtemperatur gerührt.

### 2. Herstellung der Beschichtungszusammensetzung

Die Herstellung der Beschichtungszusammensetzung erfolgt durch Vermischung der Nanosole mit hydrophob wirkenden Komponenten unmittelbar vor der Beschichtung zu einem homogenen Sol. Weiterhin kann für die Anwendung die Beschichtungszusammensetzung mit Ethanol oder Wasser weiter verdünnt werden.

### Hydrophob wirkende Komponenten A:

A1: iso-Butyltriethoxysilan (Anteil von 3%)
A2: Octyltriethoxysilan (Anteil von 3%)
A3: Hexadecyltrimethoxysilan (Anteil von 3%)
A4: Perfluorooctyltriethoxysilan (Anteil von 1%)

### 3. Anwendungen der Beschichtungszusammensetzung

Die Beschichtungszusammensetzungen werden eingesetzt zur Beschichtung von textilen Wundverbänden aus Baumwolle, Viskose und Viskose/Polyester, sowie zur Evaluierung der hydrophoben Eigenschaften auch auf Glas. Die Beschichtungen erfolgen mittels Tauchbeschichtung (Ziehgeschwindigkeit 30cm/min) oder Foulardierung (Geschwindigkeit 3m/min bei Andrücken von 2 oder 6 bar). Nach Trocknung werden die beschichteten Substrate zwischen 25°C bis 180°C thermisch nachbehandelt. Die Beurteilung hydrophober Eigenschaften erfolgt mittels Kontaktwinkelmessungen auf den beschichteten Materialien. Die Wundhaftung der beschichteten Materialien wird mittels Trennkraftmessungen zu einer Proteinlösung mit Fibrinogen evaluiert. Die Ergebnisse der Trennkraftmessungen für Proben erzeugt mittels Tauchbeschichtung sind in folgender Tabelle für einige Anwendungsbeispiele dargestellt.

| Sol | Hydrophobe Komponente | Textiles Material | Temperungstemperatur [°C] | Trennkraft [N] |
|---|---|---|---|---|
| Referenz - Textil ohne Beschicht. | --- | Viskose-Gewirke | --- | 0,40 |
| Referenz - Textil ohne Beschicht. | --- | Baumwolle- Gewebe | --- | 0,89 |
| 1 | A2 | Viskose-Gewirke | 120°C | 0,34 |
| 2 | A1 | Viskose-Gewirke | 120°C | 0,12 |
| 2 | A2 | Viskose-Gewirke | 120°C | 0,18 |
| 2 | A3 | Viskose-Gewirke | 120°C | 0,14 |
| 3 | A4 | Baumwolle- Gewebe | 60°C | 0,44 |
| 3 | A4 | Viskose-Gewirke | 60°C | 0,15 |
| 4 | --- | Baumwolle- Gewebe | 60°C | 0,59 |
| 5 | --- | Baumwolle- Gewebe | 60°C | 0,33 |
| 5 | --- | Viskose-Gewirke | 60°C | 0,25 |

### 4. Bevorzugtes Anwendungsbeispiel

Bei dem textilen Material handelt es sich um ein Flächengebilde, analog der Patentschrift DE3213673, in Form eines Wundgewirkes aus 100% Viskose mit einem Flächengewicht von 236 g/m². Das Beschichtungsmittel wird hergestellt durch Vermengen des Sols 2 mit 3% Hexadecyltrimethoxysilan als hydrophobe Komponente. Die Beschichtungsflotte ist eine 5%-ige Verdünnung des Beschichtungsmittels in Ethanol. Die Beschichtung erfolgt mittels Foulard, bestehend aus 2 Walzen in Vertikalanordnung und einem Chassis, bei einer Geschwindigkeit von 3 m/min. Der Andruck beträgt 2 bar, wodurch eine Flottenaufnahme von 207 g/m² erreicht wird. Danach lässt man das beschichtete Material 2 Stunden an der Luft hängend trocknen. Die Temperung erfolgt hängend 1 Stunde bei 120°C im Wärmeschrank. Das Beschichtungsgewicht beträgt 0,7%. Das Ergebnis der Trennkraftmessung beträgt: 0,29N.

Die Trennkraftmessungen zur Evaluierung der Wundhaftung der beschichteten Materialien erfolgen mit einer standardisierten Messmethode der Bekleidungsphysiologischen Institute Hohenstein (AW-QM-11.01/06/08.03.013). Diese Messmethode wird mit den folgenden Hilfsmitteln durchgeführt. Als Prüfling wird das beschichtete (oder imprägnierte) Textil verwendet. Als Trägermaterial dient eine Glasplatte, auf die eine Fibrinogenlösung und das Textil aufgebracht wird. Um das Ablaufen der Fibrinogenlösung beim Auftragen auf die Glasplatte zu verhindern, wird ein Gewebeband als Dichtung um den Rand der Glasplatte geklebt. Ein weiterer Streifen eines Klebebandes ("Tesa-Moll") wird zur Abdichtung auf der Glasplatte befestigt. Der textile Prüfling wird an einem der schmalen Enden mit einem elastischen Kunststoffstreifen für die später folgende Trennkraftmessung zusammengenäht.

Zur Durchführung der Trennkraftmessung wird zuerst die Fibrinogenlösung auf die horizontal ausgerichtete Glasplatte gegeben und gleichmäßig verteilt. Der Prüfling wird ohne Andruck auf die Fibrinogenlösung gelegt. Es erfolgt ein Antrocknen des Prüflings auf der Fibrinogenschicht bei Raumtemperatur über einen Zeitraum von 2 Stunden. Nach dieser Zeit werden die Prüflinge dem Normklima angepasst. Zur Bestimmung des Trennkraftwerts wird eine Zugprüfmaschine eingesetzt. Hierzu wird die Glasplatte in die untere Klemmbacke der Zugprüfmaschine und der Kunststoffstreifen in die obere Klemmbacke eingespannt. Anschließend erfolgt die Abtrennung des Prüflings von der Fibrinogenschicht. Es wird die Kraft gemessen, die benötigt wird, um den Prüfling von der Fibrinogenschicht zu trennen.

## Patentansprüche

1. Verbund aus einer Wundauflage und einer Beschichtungszusammensetzung, die aus einem Nanosol besteht, das Siliziumoxid und mindestens eine hydrophobe organische Siliziumverbindung enthält, oder einer antiadhäsiven Schicht, die ein Xerogel mit Siliziumoxid und mindestens einer hydrophoben organischen Siliziumverbindung umfasst und ein relatives Beschichtungsgewicht auf der Wundauflage im Bereich von 0.05% bis 5% aufweist.

2. Verbund nach Anspruch 1, wobei die hydrophobe organische Siliziumverbindung eine oder mehrere Verbindungen umfasst, die aus den folgenden Gruppen ausgewählt sind:
- Trialkoxysilan R¹Si(OR)₃, wobei R¹ ein Alkylrest mit 8 bis 18 Kohlenstoffatomen ist,
- Arylsilan R²Si(OR)₃ oder ein Diarylsilan R²₂Si(OR)₂, wobei R² ein Arylrest ist,
- Triphenylsilanchlorid oder t-Butyldiphenylsilanchlorid,
- hydrophob modifizierte Polysiloxane mit Alkyl- und/oder Phenylseitengruppen,
- oleophobe Verbindungen R³Si(OR)₃, wobei R³ ein perfluorierten Alkylrest ist, und
- oleophobe Polysiloxane mit perfluorierten Alkylseitenketten.

3. Verbund nach Anspruch 1 oder 2, wobei die Beschichtungszusammensetzung oder die antiadhäsive Schicht eine Epoxysilan-Verbindung enthält, so dass partiell hydrophile Eigenschaften gebildet werden.

4. Verbund nach einem der Ansprüche 1 bis 3, bei dem die Wundauflage ein textiles Flächengebilde, Schaumstoff oder Gel umfasst.

5. Verfahren zur antiadhäsiven Beschichtung einer Wundauflage, mit den Schritten:
- Herstellung einer Beschichtungszusammensetzung, die aus einem Nanosol besteht, das Siliziumoxid und mindestens eine hydrophobe organische Siliziumverbindung enthält, wobei das Siliziumoxid-haltige Nanosol durch Hydrolyse von Tetraalkoxy-silanen und mindestens einer hydrophoben organischen Siliziumverbindung in einem organischen, organisch-wässrigen oder wässrigen Lösemittel gebildet wird,
- Auftragung der Beschichtungszusammensetzung auf die Wundauflage in einer Menge, dass die antiadhäsive Schicht nach der Trocknung ein relatives Beschichtungsgewicht auf der Wundauflage im Bereich von 0.05% bis 5% aufweist, und
- Trocknung zur Bildung einer Xerogel-Schicht durch Lösungsmittelentzug.

6. Verfahren nach Anspruch 5, bei dem die Auftragung der Beschichtungszusammensetzung eine einseitige Beschichtung, eine zweiseitige Beschichtung oder eine Imprägnierung der Wundauflage umfasst.

7. Verfahren nach Anspruch 5 oder 6, bei dem die Auftragung als geschlossene Beschichtung oder Imprägnierung oder als teilweise unterbrochene Beschichtung oder Imprägnierung erfolgt.

8. Verfahren nach einem der Ansprüche 5 bis 7, bei dem nach der Trocknung eine Wärmebehandlung bei einer Temperatur zwischen 25°C bis 180°C erfolgt.

9. Verwendung einer Beschichtungszusammensetzung, die aus einem Nanosol besteht, das Siliziumoxid und mindestens eine hydrophobe organische Siliziumverbindung enthält, zur Ausrüstung von Wundauflagen, um die Adhäsion zwischen Wunde und Wundauflage zu verringern.

10. Verwendung nach Anspruch 9, bei der die hydrophobe organische Siliziumverbindung eine oder mehrere Verbindungen umfasst, die aus den folgenden Gruppen ausgewählt sind:
- Trialkoxysilan R¹Si(OR)₃, wobei R¹ ein Alkylrest mit 8 bis 18 Kohlenstoffatomen ist,
- Arylsilan R²Si (OR) ₃ oder ein Diarylsilan R²₂Si(OR)₂, wobei R² ein Arylrest ist,
- Triphenylsilanchlorid oder t-Butyldiphenylsilanchlorid,
- hydrophob modifizierte Polysiloxane mit Alkyl- und/oder Phenylseitengruppen,
- oleophobe Verbindungen R³Si(OR)₃, wobei R³ ein perfluorierten Alkylrest ist, und
- oleophobe Polysiloxane mit perfluorierten Alkylseitenketten.

11. Verwendung nach einem der Ansprüche 9 oder 10, bei der die Beschichtungszusammensetzung eine Epoxysilan-Verbindung enthält und partiell hydrophile,Eigenschaften gebildet werden.

## Claims

1. A composite of a wound dressing and a coating composition, that comprises a nanosol, which contains silica and at least one hydrophobic organic silicon compound, or an anti-adhesive layer which comprises a xerogel with silica and at least one hydrophobic organic silicon compound and which has a relative coating weight on the wound dressing in the range from 0.05 % to 5 %.

2. The composite according to claim 1, wherein the hydrophobic organic silicon compound comprises one or a plurality of compounds which are selected from the following groups:
- trialkoxysilane R¹Si(OR)₃, wherein R¹ is an alkyl group having 8 to 18 carbon atoms,
- arylsilane R²Si (OR) ₃ or a diarylsilane R²₂Si (OR) ₂, wherein R² is an aryl group,
- triphenylsilane chloride or t-butyldiphenylsilane chloride,
- hydrophobically modified polysiloxanes having alkyl and/or phenyl side groups,
- oleophobic compounds R³Si (OR) ₃, wherein R³ is a perfluorinated alkyl group, and
- oleophobic polysiloxanes having perfluorinated alkyl side chains.

3. The composite according to claim 1 or 2, in which the coating composition or the anti-adhesive layer contains an epoxysilane compound, so that partially hydrophilic properties are created.

4. The composite according to one of claims 1 to 3, in which the wound dressing comprises a flat textile form, foamed plastic or gel.

5. A method for the anti-adhesive coating of a wound dressing, having the steps:
- manufacture of a coating composition which consists of a nanosol that contains silica and at least one hydrophobic organic silicon compound, wherein the silica-containing nanosol is formed by hydrolysis of tetraalkoxysilanes and at least one hydrophobic organic silicon compound in an organic, organic-aqueous or aqueous solvent,
- application of the coating composition on the wound dressing in such an amount that the anti-adhesive layer after drying has a relative coating weight on the wound dressing in the range from 0.05 % to 5 %, and
- drying by means of solvent removal to form a xerogel layer.

6. The method according to claim 5, in which the application of the coating composition comprises a single-sided coating, a two-sided coating or an impregnation of the wound dressing.

7. The method according to claim 5 or 6, in which the application is implemented as a closed coating or impregnation or as a partly discontinuous coating or impregnation.

8. The method according to one of claims 5 to 7, in which after the drying a heat treatment is carried out at a temperature between 25 °C and 180 °C.

9. Use of a coating composition, which consists of a nanosol that contains silica and at least one hydrophobic organic silicon compound, for the treatment of wound dressings, so as to decrease the adhesion between wound and wound dressings.

10. The use according to Claim 9, in which the hydrophobic organic silicon compound comprises one or a plurality of compounds which are selected from the following groups:
- trialkoxysilane R¹Si(OR)₃, wherein R¹ is an alkyl group having 8 to 18 carbon atoms,
- arylsilane R²Si(OR)₃ or a diarylsilane R²₂Si(OR)₂, wherein R² is an aryl group,
- triphenylsilane chloride or t-butyldiphenylsilane chloride,
- hydrophobically modified polysiloxanes having alkyl and/or phenyl side groups,
- oleophobic compounds R³Si (OR) ₃, wherein R³ is a perfluorinated alkyl group, and
- oleophobic polysiloxanes having perfluorinated alkyl side chains.

11. The use according to one of claims 9 or 10, in which the coating composition contains an epoxysilane compound and partially hydrophilic properties are created.

## Revendications

1. Structure composite constituée d'un pansement et d'une composition de revêtement, faite d'un nanosol qui contient de l'oxyde de silicium et au moins un composé de silicium organique hydrophobe, ou d'une couche antiadhésive qui comprend un xérogel avec de l'oxyde de silicium et au moins un composé de silicium organique hydrophobe, et présentant un poids relatif du revêtement sur le pansement dans la plage de 0,05% à 5%.

2. Structure composite selon la revendication 1, dans laquelle le composé de silicium organique hydrophobe comprend un ou plusieurs composés choisis dans les groupes suivants :
- trialcoxysilane R¹Si(OR)₃, où R¹ est un reste alkyle de 8 à 18 atomes de carbone,
- arylsilane R²Si (OR) ₃ ou diarylsilane R²₂Si (OR) ₂, où R² est un reste aryle,
- chlorure de triphénylsilane ou chlorure de t-butyl-diphénylsilane,
- polysiloxanes à modification hydrophobe portant des groupes latéraux alkyle et/ou phényle,
- composés oléophobes R³Si (OR) ₃, où R³ est un reste alkyle perfluoré, et
- polysiloxanes oléophobes portant des chaînes latérales alkyle perfluoré.

3. Structure composite selon la revendication 1 ou la revendication 2, dans laquelle la composition de revêtement ou la couche antiadhésive contient un composé d'époxysilane de façon à former des propriétés partiellement hydrophiles.

4. Structure composite selon l'une des revendications 1 à 3, dans laquelle le pansement comprend une structure plane textile, une mousse ou un gel.

5. Procédé de revêtement antiadhésif d'un pansement, comprenant les étapes consistant à :
- préparer une composition de revêtement faite d'un nanosol qui contient de l'oxyde de silicium et au moins un composé de silicium organique hydrophobe, le nanosol contenant de l'oxyde de silicium étant formé par hydrolyse de tétraalcoxysilanes et d'au moins un composé de silicium organique hydrophobe dans un solvant organique, organo-aqueux ou aqueux,
- déposer la composition de revêtement sur le pansement en une quantité telle que la couche antiadhésive présente après séchage un poids de revêtement relatif sur le pansement dans la plage de 0,05% à 5%, et
- effectuer un séchage pour former une couche de xérogel par élimination du solvant.

6. Procédé selon la revendication 5, dans lequel le dépôt de la composition de revêtement comprend un revêtement simple face, un revêtement double face ou une imprégnation du pansement.

7. Procédé selon la revendication 5 ou la revendication 6, dans lequel le dépôt se fait sous la forme d'un revêtement ou d'une imprégnation fermé(e) ou sous la forme d'un revêtement ou d'une imprégnation partiellement interrompu(e).

8. Procédé selon l'une des revendications 5 à 7, dans lequel, après le séchage, un traitement thermique est effectué à une température allant de 25 °C à 180 °C.

9. Utilisation d'une composition de revêtement faite d'un nanosol qui contient de l'oxyde de silicium et au moins un composé de silicium organique hydrophobe pour revêtir des pansements afin de réduire l'adhérence entre la plaie et le pansement.

10. Utilisation selon la revendication 9, dans laquelle le composé de silicium organique hydrophobe comprend un ou plusieurs composés choisis dans les groupes suivantes :
- trialcoxysilane R¹Si(OR)₃, où R¹ est un reste alkyle de 8 à 18 atomes de carbone,
- arylsilane R²Si(OR)₃ ou diarylsilane R²₂Si(OR)₂, où R² est un reste aryle,
- chlorure de triphénylsilane ou chlorure de t-butyl-diphénylsilane,
- polysiloxanes à modification hydrophobe portant des groupes latéraux alkyle et/ou phényle,
- composés oléophobes R³Si (OR) ₃, où R³ est un reste alkyle perfluoré, et
- polysiloxanes oléophobes' portant des chaînes latérales alkyle perfluoré.

11. Utilisation selon l'une des revendications 9 ou 10, dans laquelle la composition de revêtement contient un composé d'époxysilane et des propriétés partiellement hydrophiles sont formées.
